# EUROPÄISCHE PATENTANMELDUNG

(11) **EP 1 005 007 A1**
(43) Veröffentlichungstag der Anmeldung: **31.05.2000**
(21) Anmeldenummer: 99122640.8
(22) Anmeldetag: 13.11.1999
(51) Int. Cl.: G09B 19/14, G09B 9/052, A61B 3/024, G02C 7/02

(54) **Vorrichtung zur Simulation des Gesichtsfeldes eines Probanden unter Drogeneinfluss**

(30) Priorität: 25.11.1998 DE 29821082 U
(71) Anmelder: Formann, Martin, 86159 Augsburg (DE)
(72) Erfinder: Schneider, Hubert, 86161 Augsburg (DE)
(74) Vertreter: Charrier, Rolf, Dipl.-Ing.

(57) **Zusammenfassung**

Die Erfindung betrifft eine Vorrichtung im wesentlichen bestehend aus einer Brillenfassung mit Gläsern (1). Bekannte derartige Vorrichtungen sind nur unzureichend geeignet, das Sehen unter Drogeneinfluß zu simulieren.

Die Aufgabe, eine Vorrichtung zur Simulation des Gesichtsfeldes eines Probanden unter Drogeneinfluß so weiterzubilden, daß eine realistische Simulation ermöglicht wird, wird dadurch gelöst, daß die Gläser Gleitsichtgläser (1) sind, die in ihrem unteren Bereich einen positiveren Dioptrienwert aufweisen als in ihrem oberen Bereich und hierdurch in ihrem linken unteren und rechten unteren Bereich (2,3) eine astigmatische Verzeichnung aufweisen und die Gläser (1) innerhalb der Brillenfassung so zentriert sind, daß die Bereiche astigmatischer Verzeichnung (2,3) sich über mindestens die Hälfte der Höhe der Gläser (1) erstrecken.

## Beschreibung

Die Erfindung betrifft eine Vorrichtung zur Simulation des Gesichtsfeldes eines Probanden unter Drogeneinfluß nach dem Oberbegriff des Anspruchs 1.

Insbesondere in Fahrschulen zur Vorbereitung auf die Führerscheinprüfung oder in Seminaren für auffällige Verkehrsteilnehmer zur Vorbereitung auf die sogenannte medizinisch-psychologische Untersuchung nach Führerscheinentzug hat es sich als wichtig erwiesen, trinkfreudigen Probanden zu demonstrieren, wie stark das Gesichtsfeld eines Menschen durch Drogeneinfluß, insbesondere Alkoholeinfluß eingeschränkt wird. Diese Demonstrationen sind deswegen so wichtig, weil unter Alkoholeinfluß für den Probanden keine Einschränkung des Gesichtsfeldes wahrnehmbar ist. Der Alkoholgehalt im Blut steigt auch bei schnellem Trinken relativ langsam und kontinuierlich an, so daß die allmähliche, zum sogenannten Tunnelblick führende Einschränkung des Gesichtsfeldes für den Probanden nicht wahrnehmbar ist. Die langsame, kontinuierliche Verschlechterung des Gesichtsfeldes fällt dem Probanden somit nicht auf Aus diesem Grunde haben sich auch Versuche als Fehlschläge erwiesen, bei denen den Probanden gezielt Alkohol zugeführt wurde. Nach reichhaltigem Alkoholgenuß fühlten sich die Probanden besser denn je in der Lage ein Fahrzeug zu führen.

Es wurden daher verschiedene Vorrichtungen erdacht und im Versuch in Betrieb genommen, die das durch Alkoholeinfluß eingeschränkte Gesichtsfeld simulieren sollen. Beispielsweise wurden den Probanden Scheuklappen aufgesetzt, so daß diese nicht mehr nach der Seite, sondern nur noch nach vorn sehen können. Diese abrupte Beschränkung des Gesichtsfeldes hat allerdings nicht viel mit dem Empfinden unter Alkoholeinfluß zu tun. Der sogenannte Tunnelblick läßt ja auch ein visuelles Empfinden im Seitenbereich zu, jedoch keine scharfe Wahrnehmung von Gegenständen und kein zuverlässiges Erkennen von Bewegungen im seitlichen Bereich.

Ferner sind Filmsimulatoren getestet worden, bei denen eine Autofahrt aus der Perspektive des Fahrers in einem Fernsehmonitor dargestellt wurde. Das Gesichtsfeld wird hier durch die Ausdehnung des Fernsehmonitors eingeschränkt, was in etwa zu den gleichen Nachteilen führt, wie sie die oben beschriebenen Scheuklappen haben. Der Übergang in den Seitenbereich erfolgt abrupt, und nicht kontinuierlich.

Schließlich wurden in der Praxis Tests mit Brillen durchgeführt, die mit Plangläsern besetzt und im linken und rechten Randbereich aufgerauht oder angeätzt waren. Ein Proband konnte durch das Planglas zwar in gerader Richtung ohne Beeinträchtigung sehen, das linke und rechte Seitenfeld erschien jedoch so stark verschwommen, daß Gegenstände und Bewegungen überhaupt nicht mehr erkannt werden konnten. Lediglich deutliche Farben sind mit diesen Brillen im Seitenbereich zu erkennen. Diese Brillen weisen damit den gleichen Nachteil auf, wie die oben beschriebenen Scheuklappen und der Filmsimulator. In dem angeätzten bzw. aufgerauhten Bereich ist praktisch überhaupt nichts mehr zu erkennen. Ein Proband wird deswegen -berechtigterweise- nicht glauben, daß die Simulation seinem Gesichtsfeld unter Alkoholeinfluß entspricht.

Die oben beschriebenen Vorrichtungen sind beispielsweise Gegenstand der DE 21 32 221, der US 4,979,902 und der US 4,698,022 sowie der US 5,372,504.

Es besteht daher die Aufgabe, eine Vorrichtung zur Simulation des Gesichtsfelds eines Probanden unter Drogeneinfluß so weiter zu bilden, daß eine realistische Simulation ermöglicht wird.

Gelöst wird diese Aufgabe mit dem kennzeichnenden Merkmal des Anspruchs 1. Vorteilhafte Ausgestaltungen sind den Unteransprüchen entnehmbar.

Ein Ausführungsbeispiel der Erfindung wird im folgenden unter Bezugnahme auf die begleitenden Zeichnungen näher beschrieben, welche zeigen
- **Fig. 1**: eine Draufsicht aufein Gleitsichtglas für eine erfindungsgemäße Vorrichtung; und
- **Fig. 2**: eine dreidimensionale Darstellung des Flächenastigmatismus des in Fig. 1 dargestellten Gleitsichtglases (nach Unterlagen der Firma Rodenstock).

Bei der erfindungsgemäßen Simulationsvorrichtung handelt es sich im wesentlichen um eine -nicht dargestellte- Brillenfassung, in die sogenannte Gleitsichtglaser eingesetzt sind. Gleitsichtgläser sind zur Korrektur von Sehfehlern bekannt und werden beispielsweise von der Firma Rodenstock unter der Marke "ProgressivR" vertrieben. Gleitsichtgläser sind im wesentlichen dadurch gekennzeichnet, daß sie im oberen Glasbereich einen anderen Korrekturwert als im unteren Glasbereich aufweisen. Ein Gleitsichtglas hat beispielsweise in seinem oberen Drittel einen Dioptrienwert von -1,5 dpt und in seinem unteren Drittel einen Dioptrienwert von +1,5 dpt während in seinem mittleren, horizontalen Bereich, im sogenannten Hauptdurchblickspunkt, ein Dioptrienwert von ca. 0 dpt vorliegt. Diese Gleitsichtgläser ermöglichen eine Sehhilfe für die Ferne und für die Nähe in einem einzigen Glas ohne Akkomodation.

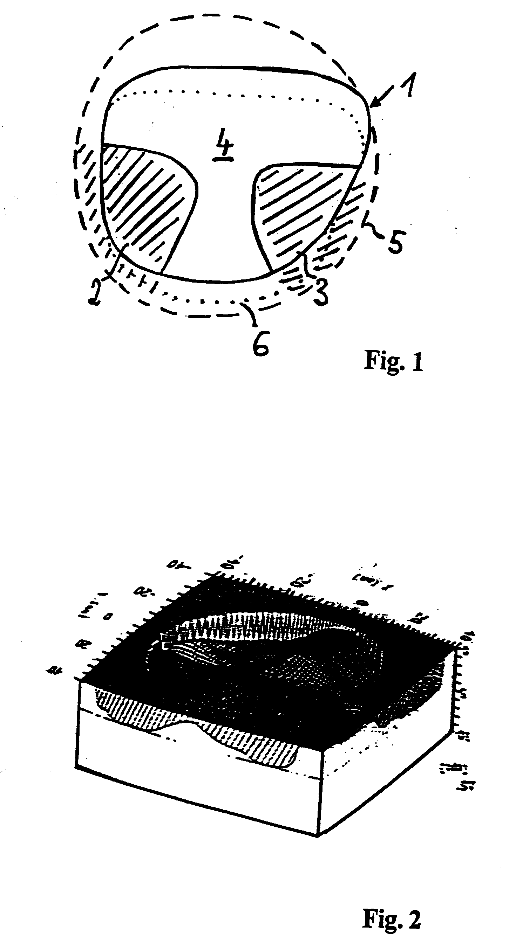

Die an sich bekannten Gleitsichtgläser 1 weisen eine für ihre Verwendung als Sehhilfe unerwünschte Eigenschaft auf, nämlich eine astigmatische Verzeichnung, welche sich etwa im linken unteren und im rechten unteren Bereich des Glases befindet. Der Bereich der astigmatischen Verzeichnung ist in Fig. 1 gestrichelt dargestellt. Der linke Bereich astigmatischer Verzeichnung trägt die Bezugszahl 2, während der rechte Bereich astigmatischer Verzeichnung die Bezugszahl 3 trägt. In der Figur nicht dargestellt ist die Tatsache, daß das Gleitsichtglas 1 in seinem oberen Drittel eine Korrektur von -1,5 dpt, in seinem mittleren Bereich im wesentlichen keine Korrektur und in seinem unteren Bereich eine Korrektur von +1,5 dpt aufweist.

In dem nicht schraffierten Bereich 4 des Gleitsichtglases 1 ist normales und scharfes Sehen möglich, während in den Bereichen 2 und 3 astigmatischer Verzeichnung nur ein undeutliches, schlierenhaftes Sehen möglich ist. Der Teil des nicht schraffierten Bereichs 4 zwischen den Bereichen 2 und 3 astigmatischer Verzeichnung ist der sogenannten Progressionskanal, durch den scharfes Sehen möglich ist.

In Fig. 1 ist der runde Rohling, aus dem das Gleitsichtglas 1 gefertigt wurde mit der Bezugszahl 5 gekennzeichnet. Die Bereiche astigmatischer Verzeichnung sind auch im Rohling 5 schraffiert dargestellt.

In einer vorteilhaften Ausgestaltung der Erfindung wird das Gleitsichtglas 1 nicht in der dargestellten Weise aus dem Rohling 5 ausgeschnitten, sondern aus dem unteren Bereich des Rohlings 5, was zu der punktierten Kontur 6 des Gleitsichtglases führt. Es ist erkennbar, daß ein derartiges Gleitsichtglas mit der Kontur 6 größere, nämlich nach oben verschobene Bereiche astigmatischer Verzeichnung 2 und 3 aufweist.

Die astigmatische Verzeichnung des Rohlings 1 wird aus Fig. 1 besonders deutlich, die eine dreidimensionale Wiedergabe des Flächenastigmatismus eines derartigen Rohlings zeigt. Die Achsen X und Y sind Raumkoordinaten, während die nach oben gerichtete Z-Achse ein Maß für die astigmatische Verzeichnung ist. Es ist deutlich erkennbar, daß etwa in der oberen Hälfte des Rohlings (positive Y-Achse) keinerlei astigmatische Verzeichnung vorliegt und sich von dort aus ein im wesentlichen vertikaler, durch die Mitte des Rohlings nach unten führender, verzeichnungsfreier Kanal erstreckt. Diese Gebiete entsprechen den nicht schraffierten Gebieten in Fig. 1. Im unteren Bereich des Rohlings befinden sich links und rechts des Kanals "Verzeichnungsgebirge", welche den Bereichen 2 und 3 astigmatischer Verzeichnung aus Fig. 1 entsprechen.

Die erfindungsgemäße Brille weist zwei Gleitsichtgläser auf, wie sie oben beschrieben wurden. Es handelt sich entweder um Standardgleitsichtgläser, bei denen die Bereiche astigmatischer Verzeichnung 2 und 3 vom unteren Glasrand nur bis etwa zur Glasmitte reichen oder vorzugsweise um Gleitsichtgläser mit einer Kontur 6, also nach unten versetzt aus dem Rohling 1 ausgeschnittene Gleitsichtgläser mit stärker betonten und weiter nach oben weisenden Bereichen 2 und 3 astigmatischer Verzeichnung. Derartige Gleitsichtgläser wurden aus Rohlingen noch nicht angefertigt, da der zur Simulation von Alkoholeinfluß erwünschte Effekt der astigmatischen Verzeichnung ansonsten unerwünscht ist.

Es ist anzumerken, daß die Vorrichtung nicht in Form einer üblichen Brille ausgestaltet sein muß, sondern auch in Form von Monokeln oder Klappgläsern, die über ein bestehendes Brillengestell geschwenkt werden. Selbstverständlich sind auch andere optische Vorsätze zur Aufnahme der Gleitsichtgläser möglich, die unter dem Begriff Brille zusammengefaßt sind. Insofern erstreckt sich der in den Ansprüchen verwendete Begriff "Brille" auch aufandere Halterungsvorrichtungen für optische Gläser.

Beim Aufsetzen einer erfindungsgemäßen Brille tritt exakt der gewünschte Simulationseffekt ein. Während der Proband nach vorn einen freien, ungestörten und klaren Blick hat ist das Gesichtsfeld nach der Seite verschleiert, verzeichnet und schlierenhaft. Allerdings erkennt der Proband auch im Seitenbereich, also in den Bereichen astigmatischer Verzeichnung 2 und 3, durchaus noch Farbe, Bewegung und Gegenstände, allerdings so verzerrt und undeutlich, wie es dem tatsächlichen Eindruck beim Fahren unter Alkohol entspricht. Der Übergang ist nicht, wie bei den eingangs angesprochenen Vorrichtungen und Verfahren abrupt, vielmehr findet ein kontinuierlicher Übergang vom Bereich schaffen Sehens zum Bereich der Verzeichnung statt, wie dies auch aus Fig. 2 hervorgeht.

Die erfindungsgemäße Vorrichtung eignet sich damit erstmals zur wirklichkeitsnahen Simulation des eingeschränkten Gesichtsfeldes unter Drogeneinfluß, insbesondere unter Alkoholeinfluß. Vorteilhafterweise ist die Vorrichtung außerdem billig, leicht zu handhaben und für größere Übungsgruppen auch in größerer Zahl verteilbar, während insbesondere der eingangs erwähnte Filmsimulator teuer, schwer handhabbar und jeweils nur einem Probanden zugänglich ist.

## Patentansprüche

1. Vorrichtung zur Simulation des Gesichtsfeldes eines Probanden unter Drogeneinfluß, im wesentlichen bestehend aus einer Brillenfassung mit Gläsern (1), **dadurch gekennzeichnet**, daß die Gläser Gleitsichtgläser (1) sind, die in ihrem unteren Bereich einen positiveren Dioptrienwert aufweisen als in ihrem oberen Bereich und hierdurch in ihrem linken unteren und rechten unteren Bereich (2, 3) eine astigmatische Verzeichnung aufweisen und die Gläser (1) innerhalb der Brillenfassung so zentriert sind, daß die Bereiche astigmatischer Verzeichnung (2, 3) sich über mindestens die Hälfte der Höhe der Gläser (1) erstrecken.

2. Vorrichtung nach Anspruch 1, **dadurch gekennzeichnet**, daß die Gläser (1) in ihrem oberen Bereich einen negativen und in ihrem unteren Bereich einen positiven Dioptrienwert aufweisen während sie in ihrem mittleren Bereich im wesentlichen keine Korrektur aufweisen.

3. Vorrichtung nach einem der voranstehenden Ansprüche, **dadurch gekennzeichnet**, daß die Gläser (1) innerhalb der Brillenfassung so zentriert sind, daß die Bereiche astigmatischer Verzeichnung (2, 3) sich über mindestens 2/3 der Höhe der Gläser (1) erstrecken.
